# EUROPEAN PATENT SPECIFICATION

(11) **EP 0 730 865 B1**
(45) Date of publication and mention of the grant of the patent: **19.12.2001**
(21) Application number: 96100360.5
(22) Date of filing: 11.01.1996
(51) Int. Cl.: A61K 31/495, A61K 31/505, A61K 31/40, A61K 31/445, A61K 31/44, A61P 25/28

(54) **Use of serotonin and dopamine receptor blockers for the treatment of mental disorders associated with cerebrovascular disease**
Verwendung von Serotonin- und Dopaminrezeptorblockern zur Behandlung von mentalen Erkrankungen verursacht durch zerebrovaskuläre Erkrankungen
Utilisation d'antagonistes de récepteurs de la sérotonine et de la dopamine pour le traitement de maladies mentales associés à les maladies cérébrovasculaires

(30) Priority: 12.01.1995 JP 319195
(43) Date of publication of application: 11.09.1996
(60) Divisional application: 01112981.4
(73) Proprietor: SUMITOMO PHARMACEUTICALS COMPANY, LIMITED, Osaka-shi, Osaka-fu 541 (JP)
(72) Inventor: Otomo, Eiichi, Tama-shi, Tokyo-to (JP)
(74) Representative: VOSSIUS & PARTNER

(56) References cited:
- EP-A- 0 600 717
- EP-A- 0 675 118
- DATABASE WPI Section Ch, Week 8024 Derwent Publications Ltd., London, GB; Class B03, AN 80-32988C XP002005785 & JP-A-55 057 572 (FERROSAN) , 29 April 1980
- DATABASE WPI Section Ch, Week 7808 Derwent Publications Ltd., London, GB; Class B02, AN 77-84676Y XP002005786 & JP-A-53 002 465 (AKZO NV) , 11 January 1978
- DATABASE WPI Section Ch, Week 8646 Derwent Publications Ltd., London, GB; Class B02, AN 86-259579 XP002005787 & JP-A-61 221 186 (JANSSEN PHARM NV) , 1 October 1986
- DATABASE WPI Section Ch, Week 8904 Derwent Publications Ltd., London, GB; Class B02, AN 88-251872 XP002005788 & JP-A-63 301 861 (PFIZER INC) , 8 December 1988
- DATABASE WPI Section Ch, Week 9117 Derwent Publications Ltd., London, GB; Class B02, AN 90-377450 XP002005789 & JP-A-03 063 263 (HOECHST ROUSSEL PHARM INC) , 19 March 1991
- DATABASE WPI Section Ch, Week 9108 Derwent Publications Ltd., London, GB; Class B02, AN 90-269293 XP002005790 & JP-A-03 007 257 (DAINIPPON PHARM KK) , 14 January 1991
- DATABASE WPI Section Ch, Week 9242 Derwent Publications Ltd., London, GB; Class B02, AN 91-312302 XP002005791 & JP-A-04 234 882 (JANSSEN PHARM NV) , 24 August 1992
- DATABASE WPI Section Ch, Week 8728 Derwent Publications Ltd., London, GB; Class B02, AN 86-259551 XP002005792 & JP-A-62 123 179 (SUMITOMO PHARM CO LTD) , 4 June 1987
- DATABASE WPI Section Ch, Week 8718 Derwent Publications Ltd., London, GB; Class B02, AN 87-036921 XP002005793 & JP-A-62 067 083 (AMERICAN HOME PROD CORP) , 26 March 1987
- DATABASE WPI Section Ch, Week 8649 Derwent Publications Ltd., London, GB; Class B02, AN 86-292965 XP002005794 & JP-A-61 236 764 (LUNDBECK H A/S) , 22 October 1986
- INT PSYCHOGERIATR, FALL 1992, 4 (2) P187-95, UNITED STATES, XP002005783 OBERHOLZER AF ET AL: "Safety and effectiveness of low-dose clozapine in psychogeriatric patients: a preliminary study."
- ARZNEIMITTELFORSCHUNG, NOV 1992, 42 (11A) P1381-90, GERMANY, XP002005784 COPPI G: "[Dihydroergocristine. A review of pharmacology and toxicology]"
- R. BERKOW ET AL. (ED.): 'The Merck Manual (fifteenth edition)', 1987, MERCK SHARP AND DOHME RESEARCH LABORATORIES, NEW JERSEY

## Description

The present invention relates to a new therapeutic agent for treating mental disorders resulting from cerebrovascular disorder.

In cerebrovascular diseases such as cerebral infarction (cerebral thrombosis and embolism) and cerebral hemorrhage, a local ischemic condition occurs in the brain, which results in a cerebral organic or functional disorder such as defluxion of the nerve cells, ventricular expansion or the like. Therefore, in patients who have cerebrovascular disease, loss of intellectual function such as depressions of mneme and memory, or various mental disorders such as emotional disturbance, loss of spontaneity, and problem behavior will develop.

Although the pathophysiological mechanism leading to the mental disorders resulting from the cerebrovascular diseases is not known, it is considered that the disorder may occur due to the development of an abnormality in intracerebral serotonin and dopamine neural functions. Thus, the following findings have been reported: ischemia causes enhanced release of serotonin or dopamine, or decreases the content thereof in the brain (Chang et al.,J. Neurochem., 60, 1483, 1993; Luthman et al., Pharmacol. Biochem. Behav., 41, 231, 1991); it increases or decreases proteins (receptors) which receive these neurotransmitters and control neurotransmission (Robinson and Starkstein, J., Geriatr. Psychiatry, 22, 1, 1989; Bracha et al., Biol. Psychiatry, 25, 265, 1989; Nagasawa et al., J. Neorosci. Res., 33, 485, 1992). As both central serotonin and dopamine nerve systems are generally known to play an important role in developing emotion and mental function, dysfunction of these systems appears to be involved in developing the mental disorders resulting from cerebrovascular disease.

Application of a dopamine-2 blocker (a typical antipsychotic agent) has been investigated as a chemotherapeutic treatment of the mental disorders resulting from cerebrovascular disease, which demonstrated that the agent was effective to some degree in the treatment of problem behavior but, in some cases, resulted in a deterioration of emotional disturbances, loss of spontaneity, and the like. Accordingly, the agent is not a satisfactory therapeutic agent for the treatment of such conditions.

### SUMMARY OF THE INVENTION

Extensive studies directed towards the treatment of various mental disorders have led to the unexpected finding that SM-9018 and Risperidon compounds which can block both serotonin-2 and dopamine-2 receptors exhibit very remarkable effects on the treatment of the mental disorders resulting from cerebrovascular disorder. The present invention is based on the above finding.

Thus, the present invention provides the use of SM-9018 or Risperidone for the preparation of a therapeutic agent for the treatment of mental disorders resulting from cerebrovascular disorders.

### DETAILED EXPLANATION OF THE INVENTION

Mental disorders that can be expected to be improved or healed with the therapeutic agent of the invention include problem behavior such as aggressive behavior, mental excitement, fugue, delirium, hallucination, delusion and the like, which are observed in patients with cerebral infarction such as upon cerebral thrombosis and embolism and cerebral hemorrhage; emotional disturbance such as anxiety, emotional tension, depressive mood, emotional incontinence, irritability, poor expression, bad humor and the like; and loss of spontaneity such as reduced positiveness, reduced expression of desires, reduced initiative in activities, reduced interest in housework, amusement and pastime, and the like.

According to the present invention, compounds which can block both serotonin-2 and dopamine-2 receptors include, the following compounds:
SM-9018, N-[4-[4-(1,2-benzisothiazol-3-yl)-1-piperazinyl]butyl]-1,2-cis-cyclohexandicarboxyimide hydrochloride having the following chemical formula: 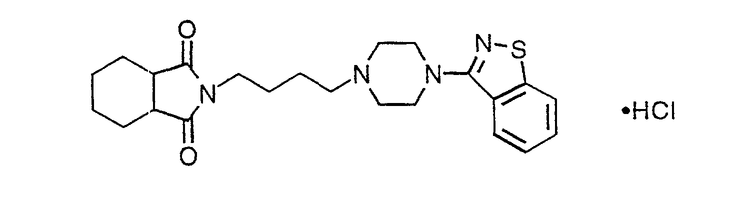 which is disclosed in Japan. J. Pharmacol. 53, 321-329 (1990); and Japanese Patent Publication (KOKAI) No.123179/1987.

Risperidone, 3-[2-[4-(6-fluoro-1,2-benzisoxazol-3-yl)piperidino]ethyl]-6,7,8,9-tetrahydro-2-methyl-4H-pyrido[1,2-a]-pyrimidin-4-one having the following chemical formula: 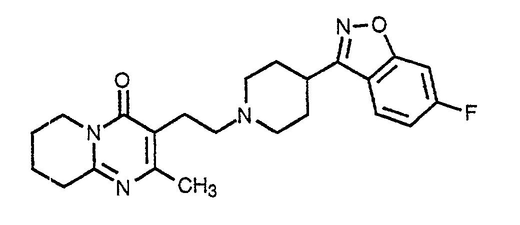 which is disclosed in Drug Data Report 1987, 9 (11), 916; and Japanese Patent Publication (KOKAI) No.221186/1986.

The foregoing compounds may be formulated to pharmaceutical formulations according to any of the methods known to those skilled in the art. For example, the compounds described above may be combined with ordinary excipients, diluents or carriers to form tablets, capsules, suspensions, powders and the like. Examples of the excipients, diluents or carriers suitable for such formulations include the followings: filling and extending agents such as starch, sugar, mannitol, and silicon derivatives; binding agents such as carboxymethyl cellulose and other cellulose derivatives, alginate, gelatin and polyvinylpyrrolidone; wetting agents such as glycerol; disintegrating agents such as sodium bicarbonate; dissolution dilating agents such as paraffine; adsorption accelerating agents such as quaternary ammonium compounds; detergents such as acetyl alcohol and glycerol monostearate; adhesive carriers such as kaoline and bentonite; and lubricating agents such as talc, calcium and magnesium stearates, and solid polyethyleneglycol.

The foregoing compounds can also be formulated as elixirs or solutions convenient for oral administration, or solutions suitable for parenteral administration (e.g. intramuscular, subcutaneous or intravenous route). Moreover, the above compounds may be formulated in a sustained release dosage form. Depending on circumstances, the compounds can be designed as a formulation which releases an active ingredient during a certain period of time, preferably only in the particular site of the intestinal tract. For example, such formulation may be covered with a coating, an envelope or a protecting matrix using a high molecular substance or wax.

The therapeutical dose of a compound of the present invention for human use, which can block both serotonin-2 and dopamine-2 receptors, will vary depending on such factors as the severity of condition of the patient, administration route and the related factors, and its determination is within the skill of a practicing physician. Typically, the acceptable effective dose for an adult is 0.05 to 500 mg/day, more typically 0.2 to 50 mg/day. Patients in need of such treatment will receive such dose once to 3 times a day, or more if necessary, for a period of time long enough to inhibit the diseases or disorders. For example, SM-9018 may be orally administered at a dose ranging from 0.5 to 10 mg/adult patient/day. More specifically, SM-9018, when orally administered, may be administered at a dosage of 1mg once to 3 times a day for 8 weeks, which may be followed by an increase in dosage to 2 mg, if the 8-week treatment failed to improve the conditions of the patients.

All of the foregoing compounds which can be used in the present invention are either already being used as medicaments or are undergoing clinical trials.

The compounds of the present invention are usually administered, in the form of pharmaceutical formulation as stated above, to patients having mental disorders resulting from cerebrovascular disorders. Specifically, such patients include those who were suffering from cerebral infarction such as cerebral thrombosis and embolism, and cerebral hemorrhage, whose symptoms are now stable for a month or more after onset, and yet who are still suffering from either problem behavior such as aggressive behavior, mental excitement, fugue, delirium or the like, emotional disturbance such as anxiety, emotional tension, depressive mood, emotional incontinence, irritability or the like, reduced spontaneity, hallucination and delusion, sleeping disorders, or somatic complaints such as the head ache, the head feeling heavy, hypochondriacal complaints, anorexia or the like. The preferable mode of administration is oral administration.

The above-listed compounds used in the present invention can be prepared according to the methods described in the publications mentioned above. For example, SM-9018 is described as Compound No.8 in Japanese Patent Publication (KOKAI) No.12379/1987, on page 21, and it may be prepared according to working examples therein.

The present invention will be illustrated in more detail with reference to the following preparations, formulations and examinations. However, they are representative only.

### Preparation 1 Synthesis of SM-9018

A mixture of 2.37 g (8.22 mmol) of N-(4-bromobutyl)-1,2-cis-cyclohexandicarboxyimide, 1.5 g (6.84 mmol) of 1-(1,2-benzisothiazol-3-yl)piperazine, 1.13 g (8.21 mmol) of potassium carbonate, 0.113 g (0.68 mmol) of potassium iodide and 25 ml of dry dimethylformamide (DMF) was heated in a warm bath at 90-100°C for 7 hours with stirring. The mixture was filtered to remove the solids, and the filtrate was concentrated under vacuum. The resultant oily residue (4.1 g) was purified by silica gel chromatography to give 2.48 g (5.81 mmol, 84.9%) of N-[4-[4-(1,2-benzisothiazol-3-yl)-1-piperazinyl]butyl]-1,2-cis-cyclohexandicarboxyimide. It was converted to a hydrochloride salt according to a conventional process, recrystallized in isopropyl alcohol to give SM-9018 [N-[4-[4-(1,2-benzisothiazol-3-yl)-1-piperazinyl]butyl]-1,2-cis-cyclohexandicarboxyimide hydrochloride]. mp. 184-185°C.

### Formulation 1 Gelatin capsules

Using the following ingredients, a hard gelatin capsule is formulated.

| Ingredients | Amount (mg/capsule) |
|---|---|
| SM-9018 | 1 |
| Starch, NF | 0-400 |
| Lactose | 0-400 |
| Magnesium stearate | 0-10 |

The above ingredients are homogeneously mixed, passed through a sieve, and then filled in a hard gelatin capsule.

### Formulation 2 Tablets

| Ingredients | Amount (mg/capsule) |
|---|---|
| SM-9018 | 1 |
| Microcrystalline cellulose | 0-200 |
| Silicon dioxide | 0-200 |
| Stearate | 0-5 |

The above ingredients are homogeneously mixed and compressed to form tablets.

### Examination 1 Clinical trial of SM-9018 for mental disorders resulting from cerebrovascular disorders.

SM-9018 was administered to patients with cerebral infarction or hemorrhage accompanied by various mental disorders described above to examine whether the mental disorders will be improved or not.
[1] Mode of administration
One mg/dosage of SM-9018 was orally administered once (Step I), twice (Step II) or 3 times a day (Step III) for up to 2 weeks after the administration was started and, when the treatment was effective, administration thereof was continued. When no effect was detected but the administration did not harm the patients, the dose of the compound administered was increased to 1 mg x 2 times/ day (Step I), 2 mg x 2 times/day (Step II), and 2 mg x 3 times/day (Step III).
[2] Administration periods: 8 weeks
[3] Results of the trials
Improvement of the mental disorders was evaluated by categorizing into 4 grades: i.e. 1) "excellent", 2) "moderate", 3) "slight", or 4) "unchanged" or other. Among the results obtained, "the number of patients categorized to 1) + 2) divided by the number of patients tested" for each symptom is summarized in the following Tables 1 to 3.
(i) Problem behaviors

**Table 1**

| | Step I | Step II | Step III |
|---|---|---|---|
| Aggressive behavior | 2/7 | 3/4 | 2/5 |
| Mental excitement | 2/14 | 4/10 | 3/8 |
| Fugue | 1/6 | 1/3 | 0/3 |
| Delirium | 1/4 | 2/5 | 1/4 |
| Total problem behavior | 2/18 | 5/10 | 3/10 |

(ii) Emotional disturbances

**Table 2**

| | Step I | Step II | Step III |
|---|---|---|---|
| Anxiety | 3/22 | 4/23 | 6/19 |
| Emotional tension | 2/18 | 3/18 | 5/15 |
| Depressive mood | 4/25 | 8/23 | 6/18 |
| Emotional incontinence | 2/13 | 3/13 | 2/10 |
| Irritability | 4/25 | 5/19 | 5/16 |
| Bad humor | 4/18 | 9/21 | 6/16 |
| Total emotional disorders | 8/33 | 11/28 | 10/20 |

(iii) Reduced spontaneity

**Table 3**

| | Step I | Step II | Step III |
|---|---|---|---|
| Reduced positiveness to an influence | 5/30 | 6/23 | 3/17 |
| Reduced expression of desires | 3/29 | 5/23 | 3/16 |
| Reduced initiative in activities | 3/30 | 5/26 | 6/17 |
| Reduced interest in housework, amusement, pastime and the like | 4/31 | 6/27 | 8/16 |
| Total loss of spontaneity | 4/32 | 8/27 | 9/18 |

(iv) Final global improvement rating

**Table 4**

| | No. of patients examined | Improvement rating | | |
|---|---|---|---|---|
| | | Excellent | Moderate | Slight |
| Step I | 35 | 2 | 5 | 20 |
| Step II | 28 | 3 | 10 | 8 |
| Step III | 20 | 6 | 7 | 3 |

According to the above global evaluation, it is evident that some improvement of those mental disorders ,including those which were judged as being "slight", was observed in about 77% of patients in Step I, about 75% in Step II, and about 80% in Step III.

## Claims

1. Use of SM-9018 or Risperidone for the manufacture of a medicament for treating mental disorders resulting from cerebrovascular disorders.

2. Use according to claim 1, wherein the amount of the compound to be administered is from 0.2 to 50 mg/adult patient/day.

3. Use according to claim 1 or 2, wherein the cerebrovascular disorder is cerebral infarction or hemorrhage.

4. Use according to any one of claims 1 to 3, wherein the mental disorder resulting from the cerebrovascular disorder is an emotional disturbance, reduced spontaneity, problem behavior, hallucination or delusion.

5. Use according to any one of claims 1 to 3, wherein the mental disorder resulting from the cerebrovascular disorder is an emotional disturbance or reduced spontaneity.

6. Use according to any one of claims 1 to 3, wherein the mental disorder resulting from the cerebrovascular disorder is reduced spontaneity.

## Patentansprüche

1. Verwendung von SM-9018 oder Risperidone für die Herstellung eines Medikaments zur Behandlung von Geisteskrankheiten, die die Folge von cerebrovaskulären Erkrankungen sind.

2. Verwendung nach Anspruch 1, wobei die Menge der zu verabreichenden Verbindung 0,2 bis 50 mg/erwachsenem Patienten/Tag entspricht.

3. Verwendung nach Anspruch 1 oder 2, wobei die cerebrovaskuläre Erkrankung ein Hirninfarkt oder eine Blutung ist.

4. Verwendug nach einem der Ansprüche 1 bis 3, wobei die Geisteskrankheit, welche aus einer cerebrovaskulären Erkrankung resultiert, eine seelische Erregung, Trägheit, Verhaltensstörung, Halluzination oder Wahnvorstellung ist.

5. Verwendung nach einem der Ansprüche 1 bis 3, wobei die Geisteskrankheit, welche aus einer cerebrovaskulären Erkrankung resultiert, eine seelische Erregung oder Trägheit ist.

6. Verwendung nach einem der Ansprüche 1 bis 3, wobei die Geisteskrankheit, welche aus einer cerebrovaskulären Erkrankung resultiert, Trägheit ist.

## Revendications

1. Utilisation de SM-9018 ou Risperidone pour la manufacture d'un médicament pour le traitement de maladies mentales résultant des maladies cérébrovasculaires.

2. Utilisation selon la revendication 1, où la quantité du composé à administrer est de 0,2 à 50 mg/adulte patient/jour.

3. Utilisation selon la revendication 1 ou 2, où la maladie cérébrovasculaire est infarctus cérébral ou hémorragie.

4. Utilisation selon l'une des revendications 1 à 3, où la maladie mentale résultant de la maladie cérébrovasculaire est un trouble affectif, spontanéité réduite, problème de comportement, hallucination ou délusion.

5. Utilisation selon l'une des revendications 1 à 3, où la maladie mentale résultant de la maladie cérébrovasculaire est un trouble affectif ou spontanéité réduite.

6. Utilisation selon l'une des revendication 1 à 3, où la maladie mentale résultant de la maladie cérébrovasculaire est spontanéité réduite.
